# EUROPEAN PATENT APPLICATION

(11) **EP 4 796 534 A1**
(43) Date of publication of application: **26.08.2026**
(21) Application number: 24880074.0
(22) Date of filing: 15.10.2024
(51) Int. Cl.: C07D 401/04, A61K 31/454, A61P 35/00

(54) **USE OF NOVEL MOLECULAR ADHESIVE**

(30) Priority: 16.10.2023 KR 20230137434
(71) Applicant: Astrogen, Inc., Daegu 41072 (KR); Korea Institute of Radiological & Medical Sciences, Seoul 01812 (KR)
(72) Inventor: HWANG, Su-Kyeong, Daegu 41072 (KR); RYU, Hyung Chul, Daegu 41072 (KR); LEE, Hae-June, Seoul 01812 (KR); LEE, Soo-Ho, Seoul 01812 (KR)
(74) Representative: Dehns
(86) International application number: PCT/KR2024/015566
(87) International publication number: WO 2025/084737

(57) **Abstract**

The present invention relates to use of a novel compound capable of functioning as a molecular adhesive for preventing or treating brain cancer.

## Description

### Technical Field

The present invention relates to use of a novel compound for prevention or treatment of brain cancer, where the novel compound may function as a molecular glue.

### Background Art

Targeted protein degradation (TPD) refers to a mechanism in which after a disease-causing factor has been translated into a protein, the corresponding protein is directly degraded and eliminated, and is represented by proteolysis-targeting chimeras (PROTAC). PROTAC consists of a protein of interest (POI) binding moiety (corresponding to the substrate protein linking region), a linker, and an E3 ligase binding moiety (corresponding to the enzyme linking region), and the core of PROTAC technology is to allow the corresponding substrate of the TPD mechanism to directly meet the E3 ligase enzyme. PROTAC consists of a protein of interest (POI) binding moiety (corresponding to a substrate protein linking region), a linker, and an E3 ligase binding moiety (corresponding to an enzyme linking region), and the core of the PROTAC technology is to allow the corresponding substrate of the TPD mechanism to directly meet the E3 ligase enzyme.

Molecular glues, which are small molecules with properties different from PROTACs, can interact with a variety of target proteins that are difficult to predict. PROTACs are divalent molecules consisting of two parts, in which one part binds to a POI and the other part binds to an E3 ligase. While PROTACs are connected by linkers and have relatively large molecular weights, molecular glues are smaller than PROTACs, and are advantageous in that they have superior pharmacological properties, higher membrane permeability, and a superior cellular uptake rate compared to PROTACs.

Various types of molecular glues have been discovered, and representative examples include thalidomide-related analogues, and lenalidomide and pomalidomide, which target the E3 ligase cereblon (CRBN).

However, thalidomide, lenalidomide, and pomalidomide have been known to have a numerical value of polar surface area (tPSA), which is close to or exceeds about 90, where the numerical value of polar surface area (tPSA) affects the passage through the blood brain barrier (BBB). This makes it difficult for these drugs to pass through the BBB.

Safety and drug tolerance are important points of consideration in the development of anti-cancer drugs, and thus there is a demand for the development of an immunomodulator having a favorable safety profile while minimizing side effects and long-term complications.

### Disclosure of Invention

### Technical Problem

An object of the present invention is to use a novel compound for prevention or treatment of brain cancer, where the novel compound may function as a molecular glue.

### Solution to Problem

[1] In an aspect of the present invention, the present invention relates to a pharmaceutical composition for prevention or treatment of brain cancer, the pharmaceutical composition containing a compound represented by following Formula 1 or Formula 2 as an active ingredient: 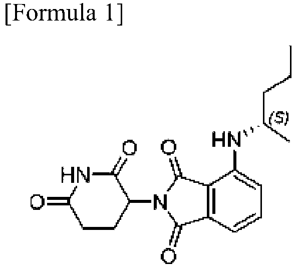 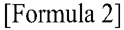 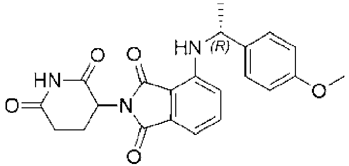
[2] In [1] above, the cancer may be a recurrent or incurable cancer.
[3] In [1] above, the cancer may be drug-resistant cancer.
[4] In [1] above, the composition may be administered by intraperitoneal administration, intravenous administration, intramuscular administration, subcutaneous administration, intradermal injection, oral administration, topical administration, intranasal injection, intrapulmonary injection, or rectal administration.
[5] In another aspect of the present invention, the present invention relates to a method for preventing or treating brain cancer in a subject, the method including a step of administering, to a subject, the compound represented by Formula 1 or Formula 2 [1] above.
[6] In another aspect of the present invention, the present invention relates to use of a compound represented by Formula 1 or Formula 2 [1] above, for preparation of a medicament for prevention or treatment of brain cancer.
[7] In still another aspect of the present invention, the present invention relates to use of a compound represented by Formula 1 or Formula 2 [1] above, for prevention or treatment of brain cancer.

### Advantageous Effects of Invention

The present invention relates to use of a novel compound that can function as a molecular glue, and the compound can be used effectively as a molecular glue in the prevention or treatment of brain cancer while minimizing side effects and long-term complications.

### Brief Description of Drawings

Fig. 1 shows the results obtained by checking an cell death effect of a compound in examples according to the present application in brain cancer cell lines (U87, U373, and SK-N-SH).
Fig. 2 shows the results of the colony-formation assay with a compound in example according to the present application in a brain cancer cell line (U87).
Fig. 3 shows the level of expression of gamma-H2AX and cleaved PARP with a compound in example according to the present application in a brain cancer cell line (U87).
Fig. 4 shows the results of the PI apoptosis assay with a compound in example according to the present application in a brain cancer cell line (U87).
Fig. 5 shows the results of the evaluation of the sub-G1 population with a compound in example according to the present application in a brain cancer cell line (U87).
Fig. 6 shows dose response curves for individual drugs.
Fig. 7 shows dose response curves in glioblastoma cell lines (U87 and U373).
Fig. 8 shows a design of in vivo experiments for a compound in examples according to the present application and the experiment results.

### Best Mode for Carrying out the Invention

The terms in this specification are used to appropriately express preferred embodiments of the present invention, and the definitions of these terms should be made based on the contents throughout this specification. Throughout the specification, when a part "includes" a certain component, this does not mean that it excludes other components, but rather that it may further include other components, unless otherwise stated.

All technical terms used in the present invention, unless otherwise defined, have the same meaning as commonly understood by an average person skilled in the art in the relevant field of the present invention. Although preferred methods and samples are described in this specification, similar or equivalent methods and samples are also included in the scope of the present invention.

In an aspect of the present invention, the present invention relates to a pharmaceutical composition for prevention or treatment of brain cancer, the pharmaceutical composition containing a compound represented by Formula 1 or Formula 2 as an active ingredient.

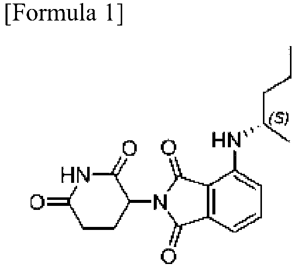

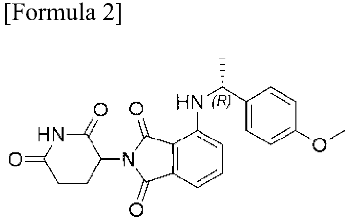

In an embodiment of the present invention, the compound represented by Formula 1 or Formula 2 may function as a molecular glue. Specifically, the compound represented by Formula 1 or Formula 2 may be a molecular glue substance that binds to a Cereblon (CRBN) protein, thereby acting to exhibit an anti-cancer effect through an immunomodulatory imide drug (IMiD) mechanism. The molecular glue is a small molecule that interacts with a target protein and has an advantage in that it has excellent pharmacological properties, membrane permeability, cell absorption rate, and blood brain barrier permeability.

In an embodiment of the present invention, the protein utilized as an endogenous target protein of immunomodulators may be a CRBN protein. CRBN proteins form an E3 ubiquitin ligase complex with damaged DNA binding protein 1 (DDB1), cullin-4A (CUL4A), and a regulator of cullin 1 (ROC1), and this complex ubiquitinates numerous other proteins. CRBN proteins are substrate receptors for CRL4, which is an E3 ubiquitin ligase, and CRL4/CRBN may regulate the intracellular homeostasis of several important intracellular proteins through ubiquitin-dependent degradation of these proteins; additionally, the CRBN proteins may directly bind to AMPK, which may cause a wide range of metabolic diseases such as cancer. Moreover, the CRBN proteins may also play a negative regulatory role in the activation of CD4+ T cells. CRBN deficiency increases CD4+ T cell activation and enhances IL-2 secretion, thereby inducing the differentiation of CD4+ T cells into Th17 cells. Enhanced T cell activation may sometimes lead to T cell-mediated autoimmune diseases, such as autoimmune encephalomyelitis and delayed-type hypersensitivity.

As used herein, the term "treatment" refers to any act by which the symptoms of a disease are improved or beneficially changed by administering a composition according to the present invention; and the term "prevention" refers to any act of inhibiting or delaying a disease by administering a composition according to the present invention; and the term "improvement" refers to any act of improving a bad condition of a disease by administering or allowing the ingestion of the composition of the present invention to a subject.

The disease targeted for "treatment," "prevention", or "improvement" of the present invention may be cancer. Cancer refers to a physiological condition in animals that is typically characterized by abnormal or uncontrolled cell growth. Cancer and cancer pathology may be associated with, for example, metastasis, interference with normally functioning surrounding cells, release of cytokines or other secreted products at abnormal levels, inhibition or enhancement of inflammatory or immunological responses, neoplasia, premalignancy, or malignancy.

In an embodiment of the present invention, the cancer may be a recurrent or incurable cancer.

In an embodiment of the present invention, the cancer may be drug-resistant cancer.

The pharmaceutical composition of the present invention may be administered in a therapeutically effective amount of the active ingredient. The therapeutically effective amount refers to the amount of drug administered that exhibits an effective effect on the prevention or treatment of cancer. The appropriate total daily dose may be determined by a practicing physician within the scope of sound medical judgment. The specific therapeutically effective dose for a particular patient may be applied differently depending on various factors including the type and degree of the response to be achieved, the type and amount of drugs to be co-administered, the specific composition including whether other preparations are used in some cases, the patient's age, weight, general health condition, sex and diet, the time period of administration, the route of administration, the duration of treatment, and similar factors well known in the medical field.

The pharmaceutical composition of the present invention may be administered to a subject or individual by any route of administration, for example, it may be administered by intraperitoneal administration, intravenous administration, intramuscular administration, subcutaneous administration, intradermal injection, oral administration, topical administration, intranasal injection, intrapulmonary injection, or rectal administration route. Preferably, the pharmaceutical composition of the present invention may be administered orally or intravenously.

In another aspect, the present invention relates to a method for preventing or treating brain cancer in a subject, the method including a step of administering, to a subject, the compound represented by Formula 1 or Formula 2 described above.

As used herein, the term "subject" is used synonymously with "individual", wherein the subject may be a mammal, for example a human, a mouse, a cow, a horse, a pig, a dog, a sheep, a goat or a cat.

Any content that overlaps with the pharmaceutical composition of the present invention described above is omitted.

In another aspect, the present invention relates to use of a compound represented by Formula 1 or Formula 2 above, for preparation of a medicament for prevention or treatment of brain cancer.

Any content that overlaps with the pharmaceutical composition of the present invention described above is omitted.

In still another aspect, the present invention relates to use of a compound represented by Formula 1 or Formula 2 above, for prevention or treatment of brain cancer.

Any content that overlaps with the pharmaceutical composition of the present invention described above is omitted.

The matters mentioned in all of the compositions, treatment methods, and uses of the present invention are equally applicable unless they are contradictory to one another.

Hereinafter, preferred embodiments are presented to help understand the present invention, but the following embodiments are only an illustration of the present invention, and it is obvious to those skilled in the art that various changes and modifications are possible within the category and technical scope of the present invention, and it is also natural that such changes and modifications fall within the scope of the appended claims.

### Mode for the Invention

### Examples

The novel compound of the present invention can be prepared according to the following reaction scheme, but is not limited thereto.

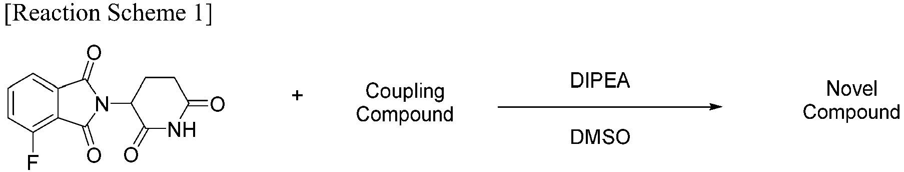

### Example 1. Synthesis of compound (AST-DT-135) coupled between 4-fluorothalidomide and (S)-penta-2-amine

As a coupling compound, (S)-penta-2-amine was used.

DMSO (60 ml), (S)-penta-2-amine (3.21 g, 0.0369 mmol, purchased from Aldrich), and N,N-diisopropylethylamine (DIPEA, 6.927 ml, 0.0398 mmol, purchased from Aldrich) (5.14 g) were added to 2-(2,6-dioxopiperidin-3-yl)-4-fluoroisodolin-1,3-dione (2-(2,6-dioxopiperidin-3-yl)-4-fluoroisoindoline-1,3-dione (4-fluorothalidomide, 10 g, 0.0362 mmol, purchased from EOS, China). The reaction mixture was stirred at 120°C for 5 hours, and then water (300 mL) was added thereto. The precipitate was filtered and washed with water (200 mL) to obtain a solid, which was subsequently recrystallized with isopropyl alcohol, subjected to vacuum drying, and confirmed the solid as a product.
Yield: 34%
Mass: 344 (M + 1)

¹H NMR (400 MHz, CDCl3): d 0.94 (3H, t), 1.24 (3H, d), 1.40 (2H, m), 1.60 (2H, m), 2.14 (1H, m), 2.80 (3H, m), 3.64 (1H, m), 4.91 (1H, m), 6.13 (1H, br s), 6.88 (1H, d), 7.06 (1H, d), 7.47 (1H, dd), 8.01 (1H, br s)

### Example 2. Synthesis of compound (AST-DT-218) coupled between 4-fluorothalidomide and (R)-1-(4-methoxyphenyl)ethylamine

As a coupling compound, (R)-1-(4-methoxyphenyl)ethylamine was used.

DMSO (80 ml), (R)-1-(4-methoxyphenyl)ethylamine (5.58 g, 0.0369 mmol), and N,N-diisopropylethylamine (DIPEA, 6.927 ml, 0.0398 mmol, purchased from Aldrich) (5.58 g)were added to 2-(2,6-dioxopiperidin-3-yl)-4-fluoroisodolin-1,3-dione (2-(2,6-dioxopiperidin-3-yl)-4-fluoroisoindoline-1,3-dione (4-fluorothalidomide, 10 g, 0.0362 mmol). The reaction mixture was stirred at 120°C for 5 hours, and then water (300 mL) was added thereto. The precipitate was filtered and washed with water (200 mL) to obtain a solid, which was subsequently recrystallized with isopropyl alcohol, subjected to vacuum drying, and confirmed the solid as a product.
Yield: 68%
Mass: 408 (M + 1)

¹H NMR (400 MHz, CDCl3) : d 1.60 (3H, d), 2.17 (1H, m), 2.85 (3H, m), 3.81 (3H, s), 4.62 (1H, m), 4.96 (1H, m), 6.61 (1H, m), 6.68 (1H, d), 6.89 (2H, d), 7.09 (1H, d), 7.28 (2H, d), 7.36 (1H, dd), 8.14 (1H, br s)

The structure and the molecular weight of each of the compounds prepared through Examples 1 and 2 described above are shown in Table 1.

**[Table 1]**

| Code | Name | Structure |
|---|---|---|
| AST-DT-135 | 2-(2,6-dioxopiperidin-3-yl)-4-(((S)-pentan-2-yl)amino)isoindoline-1,3-dione | 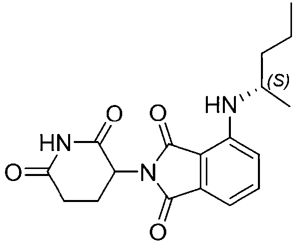 |
| | | C₁₈H₂₁N₃O₄ |
| | | Exact Mass: 343.1532 |
| | | Mol. Wt.: 343.3770 |
| AST-DT-218 | 2-(2,6-dioxopiperidin-3-yl)-4-(((R)-1-(4-methoxyphenyl)ethyl)amino)isoindoline -1,3-dione | |

### Experimental Example 1. In vitro assay

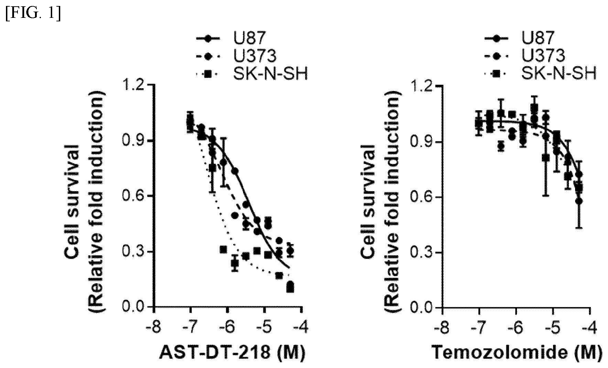

An *in vitro* experiment was carried out to check whether the compound (AST-DT-218) prepared in Example 2 was effective in the cell death in the brain cancer. First, brain cancer cell lines (U87, U373, and SK-N-SH) were cultured in the respective flasks to prepare cells that had reached the logarithmic growth stage. Then, the cells were washed with PBS, harvested with trypsin, centrifuged to adjust the cell density, and then resuspended in a suitable culture medium, and the cells were dispensed into a 96-well plate. The culture was carried out until the cells were attached to the plate and stabilized. AST-DT-218 and temozolomide, a conventional therapeutic agent for glioblastoma, were prepared and diluted at various concentrations (1/2 dilution from the highest concentration of 50 µM, 10-point). The cells in the well of the plate were treated for 48 hours with each compound at individual concentrations, and the cells were cultured to react with the compound. After the culture was completed, the number of cells was measured through the nuclear staining of the cells to evaluate the cell survival rate (Fig. 1), and IC₅₀ was calculated (Table 2).

**[Table 2]**

| IC₅₀(mM) | Brain Cancer Cell Line | | |
|---|---|---|---|
| | U87 | U373 | SK-N-SH |
| AST-DT-218 | 3.386 | 0.700 | 0.239 |
| Temozolomide | 291.2 | - | 24.570 |

Through this, it was confirmed that AST-DT-218 exhibits efficacy superior to temozolomide and induces cell death at a very low concentration

### Experimental Example 2. Colony-formation assay

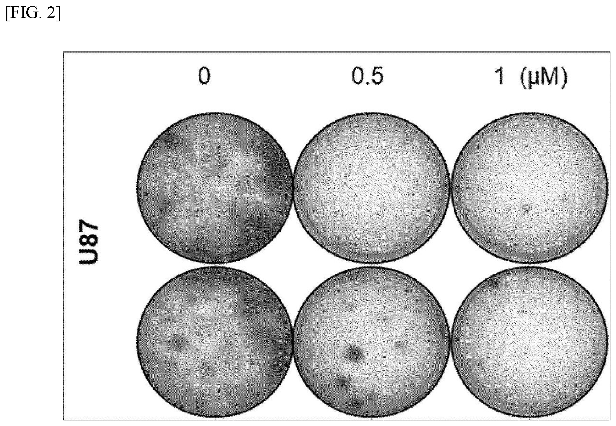

U87 cells, which are a brain cancer cell line, were cultured, the cells were separated using Trypsin-EDTA when the cells grew sufficiently, the cells were distributed uniformly on a 12-well plate, and the cells were treated with AST-DT-218 in each well after being attached. Then, the cells were cultured for about two weeks in an environment of 37°C and about 5% CO₂. When the cultivation was completed, the cells were fixed. Then, the colonies were stained with a crystal violet solution, and after the staining, the colonies were washed with PBS to remove background staining. A microscope was used to observe the colonies, and the number and size of the colonies were measured and quantified (Fig. 2).

Through this, it was confirmed that AST-DT-218 significantly reduced the formation of colonies in U87 cells.

### Experimental Example 3. DNA fragmentation and apoptosis

### Experimental Example 3-1. Western blotting

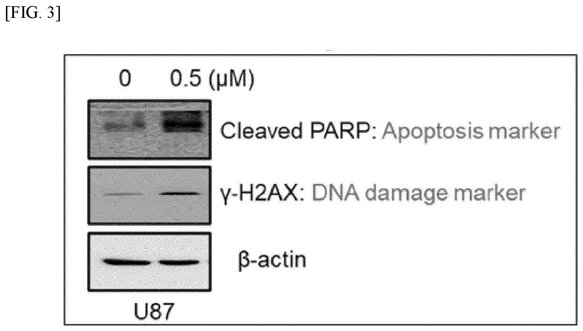

The following experiments were carried out to check whether AST-DT-218 induces DNA fragmentation and apoptosis. First, U87 cells were cultured, the cells were separated using Trypsin-EDTA when the cells grew sufficiently, the cells were plated and distributed uniformly on a 12-well plate, and the cells were treated with AST-DT-218 after being attached. Subsequently, the cells were washed with PBS, proteins were extracted using a cell lysis buffer, and the cell extract was centrifugated to collect the supernatant. Then, protein samples having the same concentration were loaded onto an SDS-PAGE gel and subjected to electrophoresis, and the proteins were transferred to a PVDF membrane. After blocking the membrane with 5% skim milk (or BSA), each of the antibodies against γ-H2AX (DNA damage marker), cleaved PARP (apoptosis marker), and beta-actin was treated at 4°C for 16 hours. The Western blotting results were analyzed to evaluate the expression levels of gamma-H2AX and cleaved PARP. Beta-actin was used as a loading control to check the consistency of the protein loading. Through this, it was confirmed that AST-DT-218 induced DNA fragmentation and apoptosis in U87 cells (Fig. 3).

### Experimental Example 3-2. Propidium iodide (PI) apoptosis assay

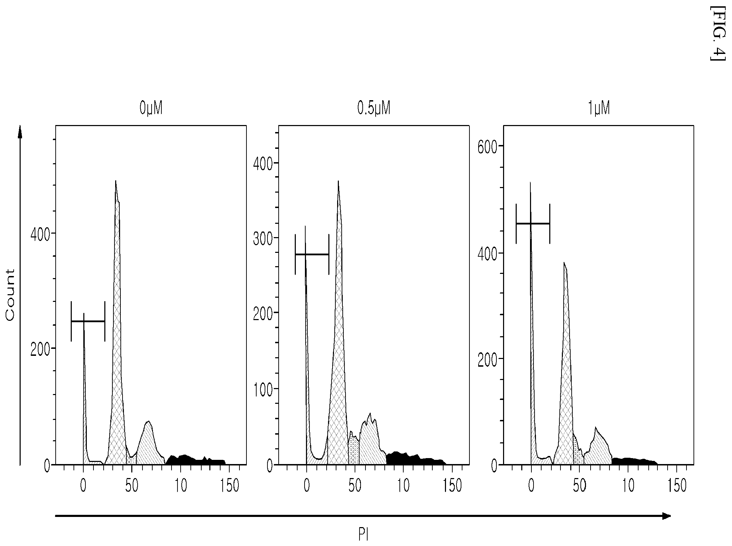

A PI apoptosis assay was carried out to check whether AST-DT-218 induces DNA fragmentation and apoptosis as in Experimental Example 3-1. A PI detection kit was prepared, propidium iodide (PI) was added to the U87 cells that had been treated with AST-DT-218, and a reaction was allowed to proceed at room temperature in a dark room for 10 minutes. Then, PI fluorescence was measured using a flow cytometry apparatus (Fig. 4). Through this, it was confirmed that AST-DT-218 induced DNA fragmentation and apoptosis.

### Experimental Examples 3-3. Measurement of sub G1 population

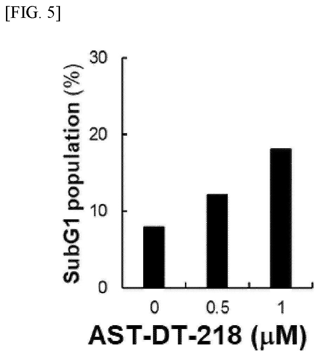

The sub G1 population was measured to check whether AST-DT-218 induces DNA fragmentation and apoptosis as in Experimental Example 3-1. First, U87 cells were treated for 48 hours with AST-DT-218 at concentrations of 0.5 µM and 1 µM, and then the morphology of the cells was observed with a microscope. The value of the sub G1 population of cells was measured using a flow cytometry apparatus (Fig. 5). Through this, it was confirmed that the value of the sub G1 population in the cell cycle increased, and it was confirmed that AST-DT-218 induced DNA fragmentation and apoptosis.

### Experimental Example 4. Dose response curve (DRC)

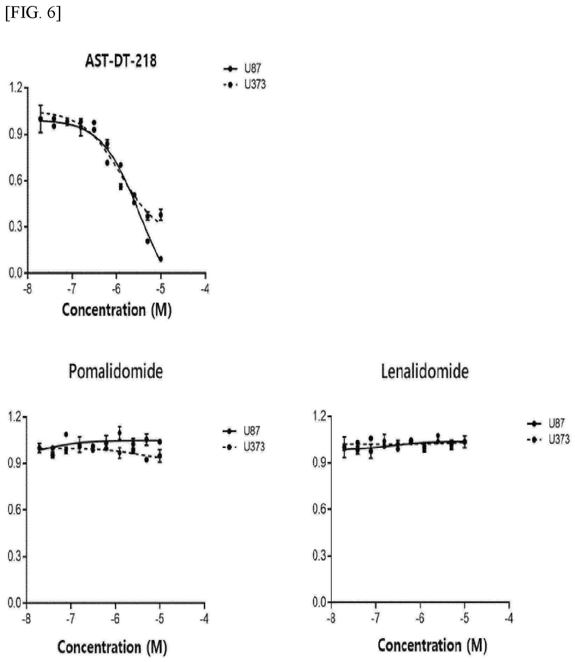

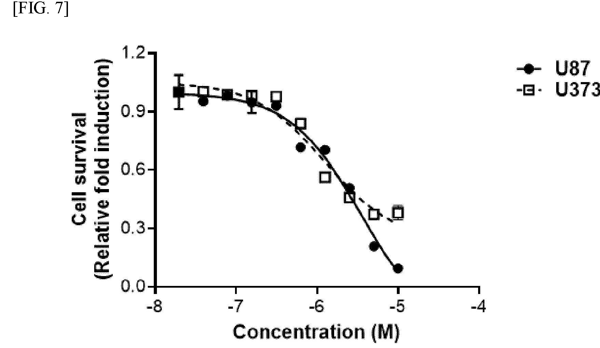

The DRC was created in order to check whether AST-DT-218 is effective in the cell death in the brain cancer. First, glioblastoma-derived brain cancer cell lines (U87 and U373) were cultured in the respective flasks to prepare cells that had reached the logarithmic growth stage. Then, the cells were washed with PBS, harvested with trypsin, centrifuged to adjust the cell density, and then resuspended in a suitable culture medium, and the cells were dispensed into a 96-well plate. Cells were cultured until they were attached to the plate and stabilized, AST-DT-218 and a control group (lenalidomide, pomalidomide) were prepared and diluted to a variety of concentrations (1/2 dilution from the highest concentration of 10 µM, 10-point). The cells in the well of the plate were treated for 48 hours with each compound at individual concentrations, and the cells were cultured to react with the compound. After the culture was completed, the number of cells was measured through the nuclear staining of the cells to evaluate the cell survival rate. The cell survival rate at each concentration was measured to collect data. Then, the DRC was created (Fig. 6 and Fig. 7), and IC₅₀ was calculated (Table 3).

**[Table 3]**

| Disease | Cell line | IC₅₀ of AST-DT-218(µM) |
|---|---|---|
| Glioblastoma | U87 | 3.363 |
| | U373 | 1.239 |

It was confirmed that as compared with lenalidomide and pomalidomide, AST-DT-218 is effective in the cell death of the brain cancer cells used in the in vitro experiment.

### Experimental Example 5. In vivo assay

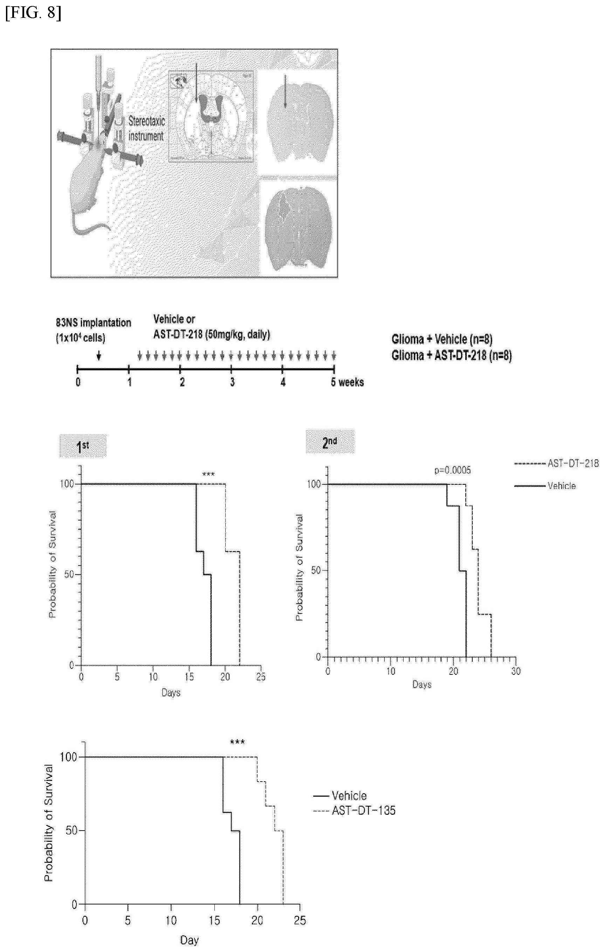

An *in vivo* experiment was carried out using a xenograft model. The experimental design and the experiment results are shown in Fig. 8. Specifically, 83NS (GSC cell line derived from a GBM patient) was used to construct an orthotopic glioblastoma model in such a way that 1 x10⁴ cells were injected into the brain of each of mice (n = 16) at a volume of 3 µl. Thereafter, a vehicle was administered to mice (n = 8), and AST-DT-218 was orally administered daily to the remaining mice (n = 8) at a concentration of 50 mg/kg for 4 weeks, which corresponds to a dose of approximately 29 mM. On the other hand, the drug to be administered was changed to AST-DT-135, and the same experiment was carried out. As a result of comparing the survival rates through the Kaplan-Meier analysis, it was confirmed that the AST-DT-135 administration group and the AST-DT-218 administration group have a significantly increased survival rate as compared with the vehicle administration group. This suggests that AST-DT-135 and AST-DT-218, which are the compounds in examples according to the present application, can exhibit effective anti-cancer effects in the glioblastoma model.

## Claims

1. A pharmaceutical composition for prevention or treatment of brain cancer, the pharmaceutical composition comprising
a compound represented by Formula 1 or Formula 2 as an active ingredient:

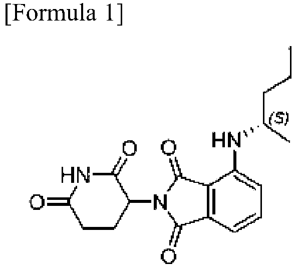

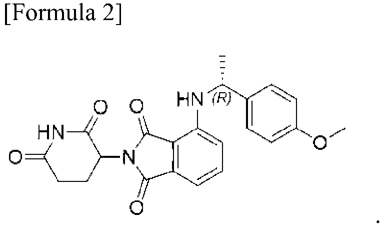

2. The pharmaceutical composition according to Claim 1,
wherein the cancer is a recurrent or incurable cancer.

3. The pharmaceutical composition according to Claim 1, wherein the cancer is drug-resistant cancer.

4. The pharmaceutical composition according to Claim 1, wherein the composition is administered through a route of intraperitoneal administration, intravenous administration, intramuscular administration, subcutaneous administration, intradermal injection, oral administration, topical administration, intranasal injection, intrapulmonary injection, or rectal administration.

5. A method for preventing or treating brain cancer in a subject, the method comprising
administering, to a subject, the compound represented by Formula 1 or Formula 2 according to Claim 1.

6. Use of a compound represented by Formula 1 or Formula 2 according to Claim 1, for preparation of a medicament for prevention or treatment of brain cancer.

7. Use of a compound represented by Formula 1 or Formula 2 according to Claim 1, for prevention or treatment of brain cancer.
